# EUROPEAN PATENT APPLICATION

(11) **EP 1 708 116 A2**
(43) Date of publication of application: **04.10.2006**
(21) Application number: 06111690.1
(22) Date of filing: 24.03.2006
(51) Int. Cl.: G06F 21/00

(54) **Method and system for unlocking a computing device**

(30) Priority: 31.03.2005 US 95677
(71) Applicant: MICROSOFT CORPORATION, Redmond, Washington 98052-6399 (US)
(72) Inventor: Chin, Peter G., Redmond, WA 98052 (US)
(74) Representative: Zimmer, Franz-Josef

(57) **Abstract**

A password locked computing device may be unlocked by coupling the locked device to a password unlocked computing device that is associated with the same user as the locked device. If the devices recognize each other as being associated with the same user, the locked computing device is automatically password unlocked without any password associated with the locked computing device being entered by the user

## Description

### Background

Digital security is major concern for many organizations. Computing devices are commonly password protected such that a device is locked when powered on to prevent unauthorized users from accessing information stored on the locked device. When synchronizing two computing devices both devices must be password unlocked before synchronization may be initiated. Some computing devices, such as personal digital assistants (PDAs), are designed for quick reference. However, the usefulness of the quick reference feature is diluted when a user is required to enter a password each time the PDA is accessed. Furthermore, many people commonly use different computing devices on a regular basis. Remembering a password for each device is burdensome for users, especially when organizations require passwords to be changed on a regular basis.

### Summary

The present disclosure is directed to a method and system for unlocking a computing device. A first computing device may be password unlocked by entering a password associated with the first computing device. A second computing device may be password locked. The second computing device may be associated with the same user as the first computing device. The first computing device couples to the second computing device. If the devices recognize each other as being associated with the same user, the second computing device is automatically password unlocked without any password associated with the second computing device being entered by the user. The computing devices may recognize each other as being associated with the same authorized user based on recognition information such as device identifiers, a key/certificate recognition partnership, or password verification.

In accordance with one aspect of the invention, a first computing device is coupled to a second computing device. The first computing device is password unlocked and the second computing device is password locked. A determination is made whether the first computing device and the second computing device recognize each other based on recognition information associated with the first computing device and the second computing device. The second computing device is unlocked when the first computing device and the second computing device recognize each other.

Other aspects of the invention include system and computer-readable media for performing these methods. The above summary of the present disclosure is not intended to describe every implementation of the present disclosure. The figures and the detailed description that follow more particularly exemplify these implementations.

### Brief Description of the Drawings

FIGURE 1 illustrates a computing device that may be used according to an example embodiment of the present invention.
FIGURE 2 illustrates functional block diagram of a system for unlocking a computing device, in accordance with at least one feature of the present invention.
FIGURE 3 illustrates an operational flow diagram illustrating a process for unlocking a computing device, in accordance with at least one feature of the present invention.

### Detailed Description of the Preferred Embodiment

The present disclosure is directed to a method and system for unlocking a computing device. A first computing device may be password unlocked by entering a password associated with the first computing device. A second computing device may be password locked. The first computing device couples to the second computing device. If the devices recognize each other as being associated with the same user, the second computing device is automatically password unlocked without any password associated with the second computing device being entered by the user.

Embodiments of the present invention now will be described more fully hereinafter with reference to the accompanying drawings, which form a part hereof, and which show, by way of illustration, specific exemplary embodiments for practicing the invention. This invention may, however, be embodied in many different forms and should not be construed as limited to the embodiments set forth herein; rather, these embodiments are provided so that this disclosure will be thorough and complete, and will fully convey the scope of the invention to those skilled in the art. Among other things, the present invention may be embodied as methods or devices. Accordingly, the present invention may take the form of an entirely hardware embodiment, an entirely software embodiment or an embodiment combining software and hardware aspects. The following detailed description is, therefore, not to be taken in a limiting sense.

### Illustrative Operating Environment

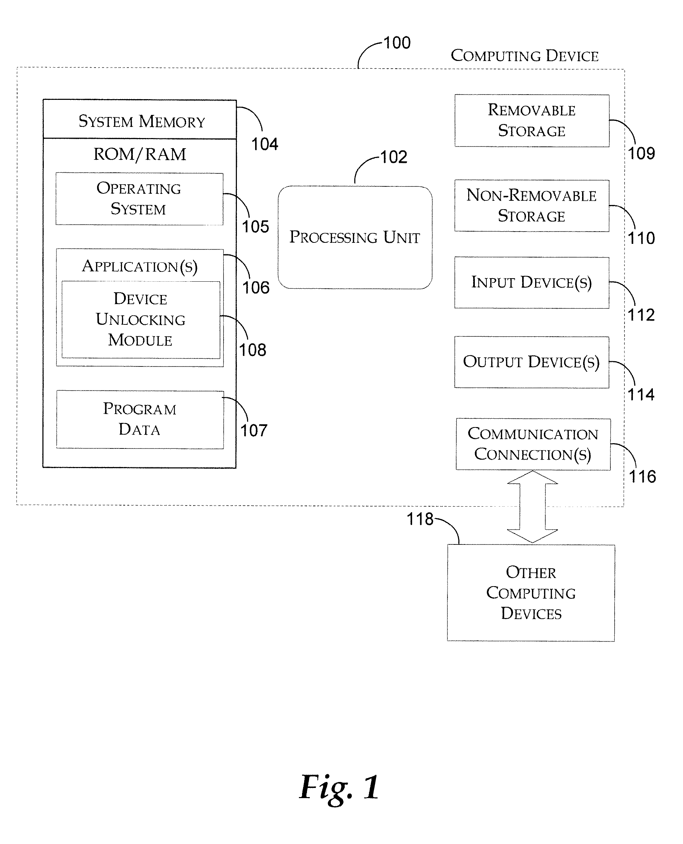

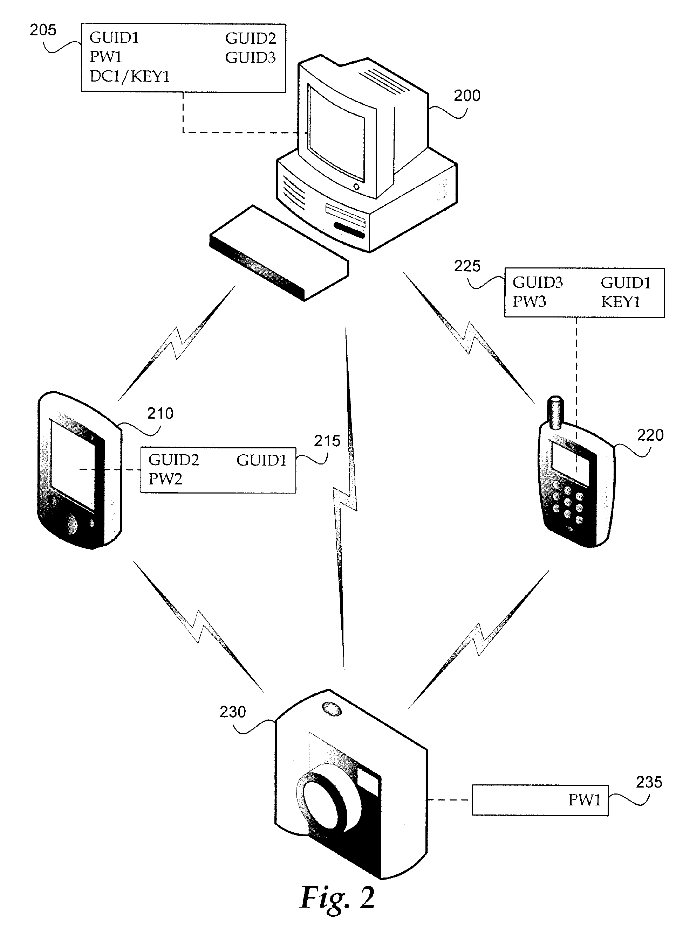

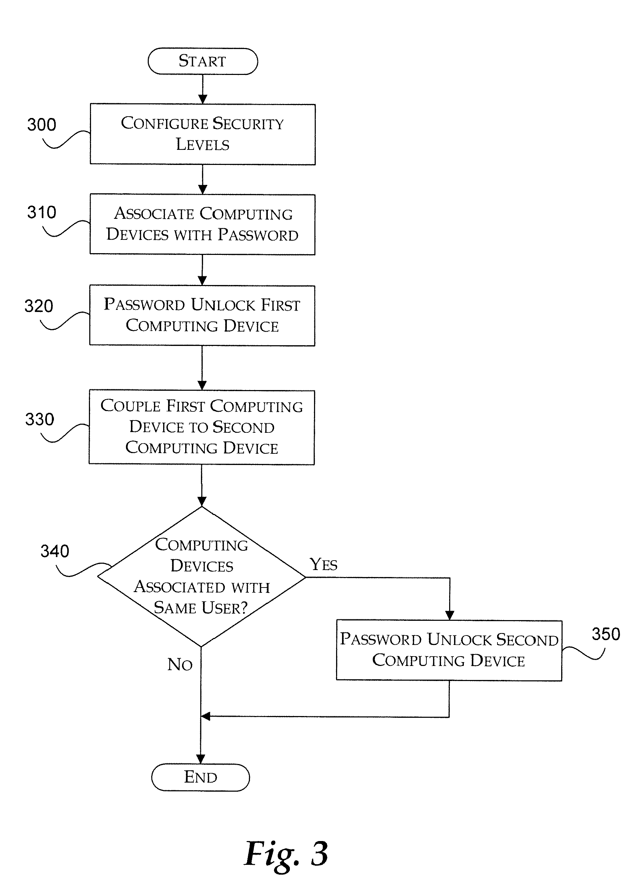

With reference to FIGURE 1, one example system for implementing the invention includes a computing device, such as computing device 100. Computing device 100 may be configured as a client, a server, a mobile device, or any other computing device that interacts with data in a network based collaboration system. In a very basic configuration, computing device 100 typically includes at least one processing unit 102 and system memory 104. Depending on the exact configuration and type of computing device, system memory 104 may be volatile (such as RAM), non-volatile (such as ROM, flash memory, etc.) or some combination of the two. System memory 104 typically includes an operating system 105, one or more applications 106, and may include program data 107. A device unlocking module 108, which is described in detail below with reference to FIGURES 2 and 3, is implemented within applications 106.

Computing device 100 may have additional features or functionality, For example, computing device 100 may also include additional data storage devices (removable and/or non-removable) such as, for example, magnetic disks, optical disks, or tape. Such additional storage is illustrated in FIGURE 1 by removable storage 109 and non-removable storage 110. Computer storage media may include volatile and nonvolatile, removable and non-removable media implemented in any method or technology for storage of information, such as computer readable instructions, data structures, program modules, or other data. System memory 104, removable storage 109 and non-removable storage 110 are all examples of computer storage media. Computer storage media includes, but is not limited to, RAM, ROM, EEPROM, flash memory or other memory technology, CD-ROM, digital versatile disks (DVD) or other optical storage, magnetic cassettes, magnetic tape, magnetic disk storage or other magnetic storage devices, or any other medium which can be used to store the desired information and which can be accessed by computing device 100. Any such computer storage media may be part of device 100. Computing device 100 may also have input device(s) 112 such as keyboard, mouse, pen, voice input device, touch input device, etc. Output device(s) 114 such as a display, speakers, printer, etc. may also be included.

Computing device 100 also contains communication connections 116 that allow the device to communicate with other computing devices 118, such as over a network. Networks include local area networks and wide area networks, as well as other large scale networks including, but not limited to, intranets and extranets. Communication connection 116 is one example of communication media. Communication media may typically be embodied by computer readable instructions, data structures, program modules, or other data in a modulated data signal, such as a carrier wave or other transport mechanism, and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared and other wireless media. The term computer readable media as used herein includes both storage media and communication media.

### Unlocking a Computing Device

The present disclosure is described in the general context of computer-executable instructions or components, such as software modules, being executed on a computing device. Generally, software modules include routines, programs, objects, components, data structures, and the like that perform particular tasks or implement particular abstract data types. Although described here in terms of computer-executable instructions or components, the invention may equally be implemented using programmatic mechanisms other than software, such as firmware or special purpose logic circuits.

FIGURE 2 illustrates a functional block diagram of a system for unlocking a computing device. A password locked computing device may be unlocked by coupling the locked device to a password unlocked computing device that is associated with the same user as the locked device. The computing device may be any device that may be password protected such as personal computer 200, personal digital assistant (PDA) 210, mobile telephone 220, and digital camera 230. Any of the computing devices may be coupled to any of the other computing devices either wirelessly or through a wired connection.

Personal computer 200 includes user interface 205, PDA 210 includes user interface 215, mobile telephone 220 includes user interface 225, and digital camera 230 includes user interface 235. Each computing device may be associated with recognition information such as a device identifier (e.g., a globally unique identifier (GUID)) and a password. For example, personal computer 200 is associated with GUID1 and password PW1, PDA 210 is associated with GUID 2 and password PW2, and mobile telephone 220 is associated with GUID3 and password PW3. Two computing devices may negotiate a recognition partnership such as public key encryption and digital certificate partnership. For example, personal computer 205 is associated with a digital certificate/key pair (DC1/KEY1). A recognition partnership may be established when personal computer transfers KEY1 to another computing device (e.g., mobile telephone 220).

At least two computing devices may be synchronized such that shared information stored on the computing devices is kept up-to-date. For example, PDA 210 may synchronize with personal computer 200 when PDA 210 is placed in a docking station that is coupled to personal computer 200. However, synchronization may not be initiated until both computing devices are password unlocked.

The recognition information associated with the computing devices may be linked such that a locked computing device may be unlocked by synchronizing with an unlocked computing device. For example, a user may unlock PDA 210 by entering password PW2. Personal computer 200 is password locked. The user may enter the vicinity of personal computer 200 with PDA 210. PDA 210 may be wirelessly coupled to personal computer 200. PDA 210 may recognize personal computer 200 as being associated with the same authorized user based on the recognition information. Thus, PDA 210 causes personal computer 200 to become automatically password unlocked without the user having to directly enter a password (i.e., PW1) for personal computer 200.

In one embodiment, computing devices may recognize each other as being associated with the same authorized user through the device identifiers. Device identifiers are commonly used in synchronization programs such as ActiveSync® developed by the Microsoft Corporation of Redmond, Washington. A synchronization partnership may be established between two computing devices when the devices are first coupled together. For example, PDA 210 may be placed in a docking station coupled to personal computer 200 for the first time (e.g., during a set-up operation). PDA 210 and personal computer 200 negotiate a recognition partnership. In one embodiment, the recognition partnership is established when the device identifier associated with PDA 210 (i.e., GUID2) is transferred to personal computer 200, and the device identifier associated with personal computer 200 (i.e., GUID1) is transferred to PDA 210. A synchronization program on each computing device stores the received GUID to manage the type of data to synchronize with the corresponding computing device. The GUIDs enable the computing devices to recognize each other as being associated with the same authorized user when the computing devices are subsequently coupled together.

In another embodiment, computing devices may recognize each other as being associated with the same authorized user through public key encryption and digital certificate technology (e.g., S/MIME). A user may establish a key/certificate recognition partnership between personal computer 200 and mobile telephone 220 as discussed above. KEY1 may be transferred from personal computer 200 to mobile telephone 220 when personal computer 200 is coupled to mobile device 220 (e.g., during synchronization). Only the computing devices that are associated with the same key/certificate partnership may synchronize with (or otherwise couple to) personal computer 200. For example, mobile telephone 220 may attempt to synchronize with personal computer 200. The key stored on mobile telephone 220 (e.g., KEY1) must match the corresponding digital certificate associated with personal computer 200 (e.g., DC1) before personal computer 200 may be password unlocked. If the key does not match the corresponding certificate, the user is not granted access to personal computer 200. The key may not match the certificate if the user associated with mobile telephone 220 is not authorized to access personal computer 200 (e.g., because mobile telephone 220 has never been coupled to personal computer 200). In one embodiment, the key may not match the certificate if the key/certificate pair is outdated. In another embodiment, the key/certificate may automatically change after a predetermined time interval has elapsed (e.g., thirty days). Changing values associated with the key/certificate partnership minimizes security breaches.

In yet another embodiment, computing devices may recognize each other as being associated with the same authorized user through password verification. Password verification may be useful when there is no synchronization or key/certificate technology available between computing devices. When a computing device is first connected to personal computer 200 (e.g., during a set-up process) the user is prompted to establish security settings for the computing device. For example, the user may couple digital camera 230 to personal computer 200. Digital camera 230 may not be enabled to recognize other computing devices through device identifiers or key/certificate partnerships. The password that is associated with personal computer 200 (i.e., PW1) may be transferred to digital camera 230. Thus, personal computer 200 may recognize digital camera 230 based on password PW1 when the two devices are subsequently coupled together. Other examples of password verification may apply to biometric sensors, voice recognition, and physical keys such as a cardkey reader.

In one embodiment, the password associated with personal computer 200 may be linked with an authorized user's log-in credentials for accessing a server that is linked to personal computer 200. If the user changes the password associated with personal computer 200, the corresponding server password may also be changed. In another embodiment, any time a password for a computing device is changed passwords corresponding to linked computing devices may also be updated.

User interfaces 205, 215, 225, 235 may be shared between the computing devices for managing the computing devices and enabling password sharing. For example, user interface 205 includes the recognition information associated with personal computer 200 (e.g., GUID1, PW1, DC1/KEY1) and the recognition information associated with recognized computing devices (e.g., GUID1, GUID3).

A user may interact with the user interface to configure settings associated with password locking/unlocking for the corresponding computing device. For example, the user may establish a setting that causes two computing devices to become automatically password locked when the computing devices are decoupled. In another example, the user may establish settings that cause only one (or neither) computing device to become password locked when the computing devices are decoupled. In yet another example, the user may configure settings to manually lock a computing device while the device is still coupled to another computing device.

FIGURE 3 illustrates an operational flow diagram illustrating a process for unlocking a computing device. The process begins at a start block where a first computing device and a second computing device are password locked. In one embodiment, the computing devices may negotiate a recognition partnership (e.g., a synchronization partnership or a key/certificate partnership) when the devices are first coupled together.

Security levels associated with the first and the second computing devices may be configured at block 300. For example, a user may establish whether both devices, only one device or neither device remains password unlocked after the computing devices are decoupled. In another example, the user may set a time interval for how frequently a password or a key/certificate pairing is updated. In one embodiment, the user may establish the type of recognition information used to determine whether the computing devices recognize each other (e.g., device identifiers, key/certificates pairings, password verification, etc.) In another embodiment, security levels are configured using a user interface that is shared between the first computing device and the second computing device.

Moving to block 310, a password is associated with the computing devices. In one embodiment, the user assigns one password to the first computing device and another password to the second computing device. Proceeding to block 320, the first computing device is password unlocked. The first computing device may be password unlocked when the user enters the password that is associated with the first computing device.

Advancing to block 330, the first computing device is coupled to the second computing device. The connection may be made either wirelessly or through a wired connection. For example, a PDA may be connected to a personal computer via a synchronization docking station.

Transitioning to decision block 340, a determination is made whether the first computing device and the second computing device recognize each other as being associated with the same user. The determination may be made based on the recognition information established by the user.

In one embodiment, the computing devices recognize each other as being associated with the same user based on corresponding device identifiers. For example, GUIDs associated with each computing device may be used to determine whether the devices have been previously coupled together (e.g., when negotiating a synchronization partnership). If the GUID associated with the first computing device corresponds to the GUID associated with the second computing device then the computing devices recognize each other as being associated with the same user. If the GUID associated with the first computing device does not correspond to the GUID associated with the second computing device then the devices do not recognize each other as being associated with the same user.

In another embodiment, the computing devices recognize each other as being associated with the same user based on successful key/certificate matching. For example, a key associated with the first computing device is transferred to the second computing device. If the key matches the certificate associated with the second computing device then the computing devices recognize each other as being associated with the same user. If the key does not match the certificate then the devices do not recognize each other as being associated with the same user.

In yet another embodiment, the computing devices recognize each other as being associated with the same user based on password verification. For example, a password associated with the first computing device may be associated with the second computing device when the devices are first coupled together. The computing devices may recognize each other as being associated with the same user based on the password when the devices are subsequently coupled together. Other examples of password verification may apply to biometric sensors, voice recognition, and physical keys such as a cardkey reader. If the computing devices do not recognize each other as being associated with the same user, processing terminates at an end block. If the computing devices recognize each other as being associated with the same user, processing continues at block 350.

Continuing to block 350, the second computing device is automatically password unlocked without any information associated with a password for the second computing device being entered by the user. The computing devices may then be decoupled. Processing continues in accordance with the configured security levels. For example, one or both devices may remain password unlocked, or both devices may be password locked. In another example, the user may be prompted to change the passwords associated with the devices after a predetermined period of time has elapsed. Processing then continues at the end block.

The above specification, examples and data provide a complete description of the manufacture and use of the composition of the invention. Since many embodiments of the invention can be made without departing from the spirit and scope of the invention, the invention resides in the claims hereinafter appended.

## Claims

1. A computer-implemented method for unlocking a computing device, comprising:
coupling a first computing device to a second computing device, wherein the first computing device is password unlocked and the second computing device is password locked;
determining whether the first computing device and the second computing device recognize each other based on recognition information associated with the first computing device and the second computing device; and
unlocking the second computing device when the first computing device and the second computing device recognize each other.

2. The computer-implemented method of Claim 1, wherein the determining further comprises determining whether a user that is associated with the first computing device is the same user that is associated with the second computing device.

3. The computer-implemented method of Claim 1, wherein the unlocking further comprises automatically unlocking the second computing device without receiving a user input corresponding to a password associated with the second computing device.

4. The computer-implemented method of Claim 1, wherein the coupling further comprises synchronizing the first computing device and the second computing device.

5. The computer-implemented method of Claim 1, further comprising negotiating a recognition partnership between the first computing device and the second computing device when the first computing device is coupled to the second computing device, wherein the recognition partnership is based on the recognition information.

6. The computer-implemented method of Claim 1, wherein the recognition information is associated with device identifiers that correspond to the first computing device and the second computing device.

7. The computer-implemented method of Claim 1, wherein the recognition information is associated with a public key and digital certificate recognition partnership established between the first computing device and the second computing device.

8. The computer-implemented method of Claim 1, wherein the recognition information is associated with a password shared between the first computing device and the second computing device.

9. The computer-implemented method of Claim 1, further comprising locking the first computing device and the second computing device when the second computing device is decoupled from the first computing device.

10. The computer-implemented method of Claim 1, further comprising locking the first computing device when the second computing device is decoupled from the first computing device.

11. The computer-implemented method of Claim 1, wherein the coupling further comprises coupling the first computing device to the second computing device through a wired connection.

12. The computer-implemented method of Claim 1, wherein the coupling further comprises coupling the first computing device to the second computing device through a wireless connection.

13. The computer-implemented method of Claim 1, further comprising changing the recognition information after a predetermined period of time has expired.

14. A system for unlocking a computing device, comprising:
a first computing device that is arranged to be password unlocked;
a second computing device coupled to the first computing device, wherein the second computing deice is arranged to be password locked; and
an unlocking module coupled to at least one of the first computing device and the second computing device, wherein the unlocking module is arranged to:
determine whether the first computing device and the second computing device recognize each other based on recognition information associated with the first computing device and the second computing device, and
unlock the second computing device when the first computing device and the second computing device recognize each other.

15. The system of Claim 14, wherein the unlocking module determines that the first computing device and the second computing device recognize each other by determining whether a user that is associated with the first computing device is the same user that is associated with the second computing device.

16. The system of Claim 14, wherein the unlocking module is further arranged to automatically unlock the second computing device without receiving a user input corresponding to a password associated with the second computing device.

17. The system of Claim 14, wherein the unlocking module is further arranged to negotiate a recognition partnership between the first computing device and the second computing device when the first computing device is coupled to the second computing device, wherein the recognition partnership is based on the recognition information.

18. A computer-readable medium having computer-executable instructions for unlocking a computing device, comprising:
coupling a first computing device to a second computing device, wherein the first computing device is password unlocked and the second computing device is password locked;
determining whether the first computing device and the second computing device recognize each other based on recognition information associated with the first computing device and the second computing device, wherein the recognition information indicates whether a user that is associated with the first computing device is the same user that is associated with the second computing device; and
unlocking the second computing device when the first computing device and the second computing device recognize each other.

19. The computer-readable medium of Claim 18, wherein the unlocking further comprises automatically unlocking the second computing device without receiving a user input corresponding to a password associated with the second computing device.

20. The computer-readable medium of Claim 18, further comprising negotiating a recognition partnership between the first computing device and the second computing device when the first computing device is coupled to the second computing device, wherein the recognition partnership is based on the recognition information.
